# EUROPEAN PATENT APPLICATION

(11) **EP 2 071 518 A2**
(43) Date of publication of application: **17.06.2009**
(21) Application number: 09156496.3
(22) Date of filing: 06.07.2007
(51) Int. Cl.: G06T 17/40

(54) **A method, apparatus, system and computer-readable medium for interactive shape manipulation**

(30) Priority: 14.07.2006 EP 06117197
(62) Divisional of application: 07805069.7
(71) Applicant: Koninklijke Philips Electronics N.V., 5621 BA Eindhoven (NL); Philips Intellectual Property & Standards GmbH, 20099 Hamburg (DE)
(72) Inventor: Lorenz, Cristian, 5600 AE, Eindhoven (NL); Renisch, Steffen, 5600 AE, Eindhoven (NL); Von Berg, Jens, 5600 AE, Eindhoven (NL)
(74) Representative: Schouten, Marcus Maria

(57) **Abstract**

A method and apparatus for interactively manipulating a shape of an object, comprising selecting an object to be manipulated and rendering the object in dependence of a manipulation type. The method provides a smart object adapted interaction, manipulation and visualization scheme in contrast to previous display driven schemes. The method allows efficient shape manipulation by restricting the degrees of freedom for the manipulation to the meaningful ones for a given object or object part, thus allowing to reduce e.g. a 3D interaction to a 2D interaction.

## Description

### FIELD OF THE INVENTION

This invention pertains in general to the field of Image Analysis. More particularly the invention relates to interactive shape editing and rendering for e.g. anatomical structures, and models for the measurement of geometrical properties of such structures from 2D, 3D and 4D patient data like X-ray, Computed Tomography CT, Magnetic Resonance Imaging MRI and Ultrasound US.

### BACKGROUND OF THE INVENTION

Interactive means to draw and edit graphical shapes can be found in almost all medical applications. These interactive means may be based on modifying the contours of the graphical shapes. Contours can be active or passive, open or closed, smooth, e.g. using Bezier interpolation, or composed from line segments. Contours are for instance used for modeling, segmentation and measuring tasks.

There are basically two ways to draw a contour, point-by-point or freehand. In case of a freehand contour, the contour is often sub-sampled and transformed into a smooth contour after the user finished the drawing. Both the point-by-point and freehand drawing methods create a contour consisting of a number of control points. Contour editing techniques are based on manipulating these control points. After drawing, the contour can be transformed to an active contour that optimizes its shape relative to the image.

To create three-dimensional (3D) surface models, a series of contours can be used. More precisely, one contour is used in each slice of a 3D data set, e.g. acquired by CT scanning. These contours are then combined to form the actual 3D model. Active contours have been used to find the location of the contour in the next slice in a 3D data set or in the next image in a time series, e.g. for motion tracking, automatically. Instead of using the slices of the volume it is also possible to use a set of arbitrary two-dimensional (2D) cuts through the volume to draw the contours that are combined to a 3D model.

Current shape editing methods may couple the degrees of freedom for the shape manipulation to the used display geometry. A 3D shape is for example displayed as cut-line in a reformatted 2D image allowing to select an object point on the cut-line and to move it along the image axes of the 2D image. If a motion in another direction is desired, it is required to firstly adjust the reformatted image to the respective deformation direction. Another example is the use of a 3D shape rendering and a 3D pointing devices (e.g. a 3D mouse) that allows to move a surface point in the 3 axes of the 3D rendering.

JP2002032786 discloses a device and a method for controlling processing parameters, three-dimensional model processor and program providing medium. JP2002032786 discloses a parameter controller which makes the deformation forms of a model easily predictable to a user by making the visual characteristic of a cursor itself, such as the size and color of the cursor, correspond to a processing parameter. JP2002032786 thus discloses a method, which facilitates visually the deformation process for a user, however JP2002032786 does not solve the problem regarding difficulties associated with deformation of 3D shapes, in which the user desires to change the shape in three dimensions.

A disadvantage of current methods is the large amount of interaction required to perform a shape manipulation. A large amount of time is initially spent to adjust the respective renderings that define the direction of the deformation, in addition to the interaction time required for the deformation per se.

Furthermore, the procedures are inherently tedious due to the too many degrees of freedom that need to be controlled but which are not beneficial to the shape deformation task to be accomplished.

Hence, an improved shape editing method, system, apparatus and computer readable medium would be advantageous allowing for increased flexibility, time reduction, ease of use, efficient user interaction and cost-effectiveness would be advantageous.

### SUMMARY OF THE INVENTION

Accordingly, the present invention preferably seeks to mitigate, alleviate or eliminate one or more of the above-identified deficiencies in the art and disadvantages singly or in any combination and solves at least the above mentioned problems at least partly by providing a method, apparatus, system and computer readable medium according to the appended patent claims.

According to one aspect of the invention, a method of manipulating a shape of a digital object, wherein the method comprises selecting an object to be manipulated, and interactively rendering the object in dependence of a manipulation type is provided.

According to another aspect of the invention, an apparatus for interactive digital shape manipulation for performing the method according to any one of appended claims 1-20 is provided. The apparatus comprises a selection unit for selecting an object to be manipulated; and a rendering unit for interactively rendering the object in dependence of a manipulation type.

According to yet another aspect of the invention a computer-readable medium having embodied thereon a computer program for processing by a computer is provided, the computer program comprising a shape manipulation code segment for manipulating a shape of an object. The computer program further comprises a render code segment for interactively rendering the object in dependence of a manipulation type.

### BRIEF DESCRIPTION OF THE DRAWINGS

These and other aspects, features and advantages of which the invention is capable of will be apparent and elucidated from the following description of embodiments of the present invention, reference being made to the accompanying drawings, in which:
Fig. 1 is a flowchart of a method according to an embodiment.
Fig. 2 is a series of illustrations showing the standardized deformation scheme according to an embodiment.
Fig. 3 is a series of illustrations showing the paddle-wheel like set of multi-planar image reformats according to other embodiments.
Fig. 4 is a schematic view showing an apparatus according to an embodiment.
Fig. 5 is a schematic view showing a computer-readable medium according to an embodiment.

### DESCRIPTION OF EMBODIMENTS

The description below focuses firstly on the reduction of degrees of freedom required for a 3D manipulation to a 2D mouse gesture and secondly on the provision of automated feedback to the user in terms of a suitable visualization scheme covering the region of influence of the shape manipulation.

A method is described that facilitates shape editing in the context of medical image segmentation using shape models and/or free-form deformation methods.

The following description focuses on embodiments of the present invention applicable to an image analysis system and in particular to a medical image analysis system capable of shape editing. However, it will be appreciated that the invention is not limited to this application but may be applied to many other applications, wherein shape editing of images is desired.

According to an embodiment a method for smart object adapted interaction, manipulation and visualization schemes in contrast to previous display driven schemes, is provided. The method allows efficient shape manipulation by restricting the degrees of freedom for the manipulation to the meaningful ones for a given object or object part, thus allowing to reduce e.g. a 3D interaction to a 2D interaction.

Contour, surface, and shape editing may be performed by mapping mouse movements. The mouse mapping may be related to the contour direction in the selected point. In that case, the mouse mapping may for instance be tangential and/or perpendicular to the contour. The mouse movement may also correspond to some other reference, i.e. direct mouse manipulation not related to the contour direction in the selected point, such as movement with respect to the viewing screen. In that case, the mouse mapping may be related to any other contour editing geometrically oriented mouse movement, or the mouse mapping may be completely detached from the orientation of the contour. The mouse movements may be just horizontal and vertical displacements similar to the direct mouse manipulation modes that control zoom, pan, roll, contrast and brightness.

A part of a contour (border) may also be reshaped using a displacement function with two degrees of freedom. These displacement functions map mouse movement to parameters such as the primary displacement (size and direction) and the size and shape of the region to be modified.

Furthermore, a part of the contour may be smoothened by utilizing mouse interaction to steer two parameters of the smoothening process and the affected region.

Active contours may also be used to edit a contour. Part of a 'normal' contour may be activated to follow image details. Also in this case the mouse is mapped to the affected region size and properties of the active contour. The opposite is also possible. Part of an active contour may be deactivated. In this case the mouse mappings may be used to alter part of the contour as described above.

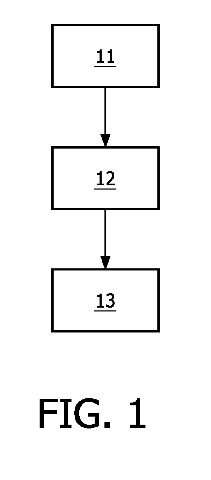

In an embodiment, according to Fig. 1, a method of manipulating a shape of an object is provided. The method comprises interactively rendering the object in dependence of a manipulation type of said manipulation. The method comprises the following,
selecting an object 11 to be manipulated,
interactively rendering 13 the object in dependence of a manipulation type 12 of the manipulation.

According to a further embodiment the object is comprised in a dataset.

In an embodiment, the dataset is a 4D, 3D or 2D image dataset.

According to an embodiment a method for manipulation of a surface patch along the surface normal of an object with amount of push/pull and radius of influence as the two relevant degrees of freedom, is provided.

According to another embodiment a method for manipulation of tube-like objects with e.g. the amount of tube bending normal to the tube axis as the two relevant degrees of freedom, is provided.

Since the interaction is object steered rather than display steered, the manipulation may be performed independently of the display type of the object (be it e.g. a 3D surface rendering or a cut-line rendering in a multi-planar reformatted image). The user selects (e.g. using a mouse pointer) an object point in whatever rendering and performs the deformation in a rendering independent standardized scheme.

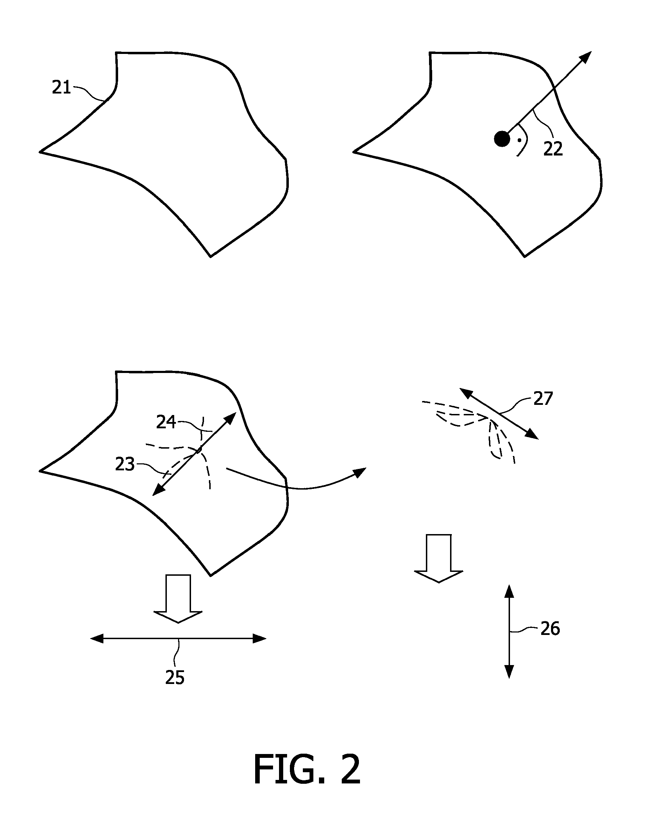

In an embodiment, according to Fig. 2, the standardized deformation scheme consists of a mouse drag with a horizontal movement 25 interpreted as pull 23 (left drag) and push 24 (right drag) of a surface patch 21 along a surface normal 22 and the vertical movement 26 interpreted as and adjustment of the influence radius 27 (e.g. increased predefined value with up-drag, decrease with down drag).

According to an embodiment, an image and object rendering method is provided. The image and object rendering method depends on the manipulation type and is used to visually control the deformation. The method selects automatically the renderings that best visualize the impact of the manipulation, e.g. by aligning the renderings with certain "main modes" of the deformation.

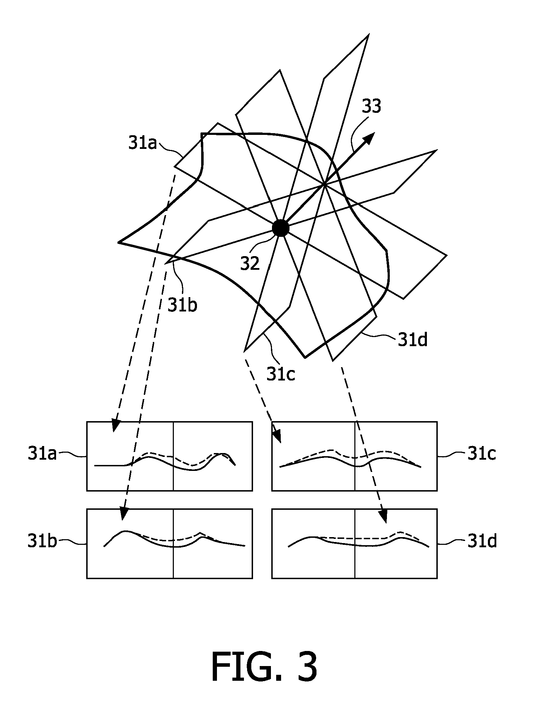

In an embodiment, according to Fig. 3, a paddle-wheel like set of multi-planar image reformats 31a-d is provided, in which the cut-lines of the deformed shape are rendered during the deformation. Position and axis and position of the paddle-wheel like set of reformats is defined by a selected surface point 32 and the surface normal 33.

According to another embodiment the paddle-wheel like set of reformats is time oriented along the axis of a tube-like structure.

In another embodiment the paddle-wheel like set of reformats may describe any of at least two 2D observation view planes.

In other embodiments the at least two 2D observation view planes has a predetermined relation.

In other embodiments the at least two 2D observation view planes has an arbitrarily determined relation.

In another embodiment other types of renderings that allow to follow the deformation scheme around the interaction point may be used.

In some embodiments the method is not restricted to the two manipulation schemes described above. Other schemes could e.g. include object cut- or join-operations or model based interaction methods where the manipulation is performed along directions predefined by a shape model.

According to an embodiment the method may be accompanied by an automated selection of the manipulation scheme, depending on the user-selected object part or a-priori knowledge as provided by a shape model.

A shape model may comprise encoding of guidance information (such as typical shape variability) for shape editing (PH002812EP1), such as the kinds of manipulation possible for the actual shape or contour. The shape model may further include sensible manipulation kinds or parameters into the shape model (e.g. derived from statistical properties of the anatomical object).

The present embodiments may be applied even if no shape model is available, e.g. in the context of 3D active objects (based on whatever explicit shape representation and after whatever expansion steps, potentially including topological changes). Furthermore the present embodiments may be applied to implicitly represented shapes (e.g. as implicit function) or to shapes defined as voxel ensembles.

In an embodiment, a method for shape editing is provided, wherein the method has the capacity to utilize a combination of model shape, control points and mouse mapping movements to edit the shape of an image.

In a further embodiment the method utilizes the paddle-wheel technique to visualize the deformation in two degrees of freedom, in several views, simultaneously.

According to other embodiments the method is capable of utilizing further kinds of manipulation, which may be defined depending on either local shape properties or certain procedures, e.g. the cutting or combining of shape objects. The manipulation may also be driven by information included in a shape model (e.g. statistically probable deformation types).

In another embodiment the method provides 3D+t shape interaction types with dedicated reduction of the spatio-temporal degrees of freedom. The motion vector of a surface point may e.g. define a selected degree of freedom.

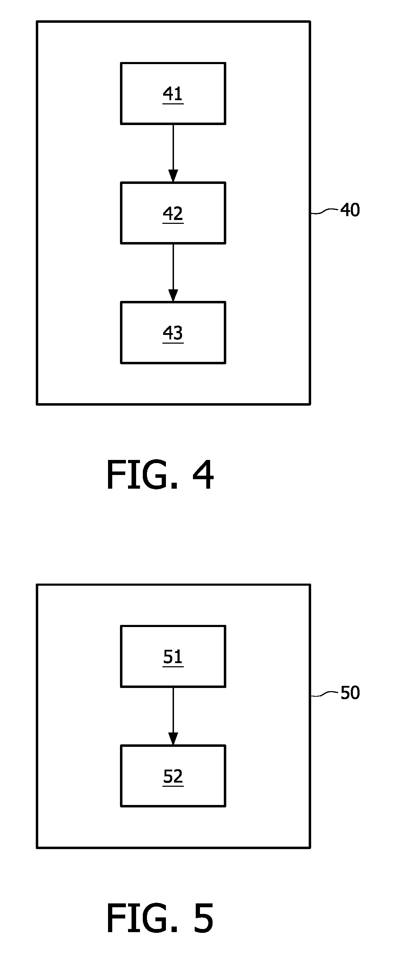

In an embodiment, according to Fig. 4, an apparatus 40 for interactive shape manipulation is provided, said apparatus 40 comprising a selection unit 41 for selecting an object to be manipulated, and a rendering unit 42 for interactively rendering the object in dependence of a manipulation type of the manipulation. The apparatus is capable of performing the method. The selection unit may be any unit normally used for such selections, such as a mouse, be it 2D or 3D, a digitizing board, etc. The rendering unit may be a hardware such as a processor with a memory. The processor could be any of variety of processors, such as Intel or AMD processors, CPUs, microprocessors, Programmable Intelligent Computer (PIC) microcontrollers, Digital Signal Processors (DSP), etc. However, the scope of the invention is not limited to these specific processors. The memory may be any memory capable of storing point information, such as Random Access Memories (RAM) such as, Double Density RAM (DDR, DDR2), Single Density RAM (SDRAM), Static RAM (SRAM), Dynamic RAM (DRAM), Video RAM (VRAM), etc. The memory may also be a FLASH memory such as a USB, Compact Flash, SmartMedia, MMC memory, MemoryStick, SD Card, MiniSD, MicroSD, xD Card, TransFlash, and MicroDrive memory etc. However, the scope of the invention is not limited to these specific memories.

In another embodiment the apparatus further comprises a display 43 for displaying the shape manipulation.

In an embodiment, the apparatus is comprised in a medical workstation or medical system. The medical workstation or medical system may also comprise other medical image equipment, such as PET, CT and MRI equipment.

In an embodiment, according to Fig. 5, a computer-readable medium having embodied thereon a computer program 50 for processing by a computer is provided. The computer program comprises a shape manipulation code segment 51 for manipulating the shape of an object. Furthermore the computer program comprises a render code segment 52 for rendering the object in dependence of a manipulation type of the manipulation.

In another embodiment the computer-readable medium comprises code segments arranged, when run by an apparatus having computer processing properties, for performing all of the method steps defined in any one of the embodiments.

The described method assigns the most meaningful degrees of freedom for shape editing to the available degrees of freedom of the input device (e.g. the 2DOF of the mouse motion). In addition, it provides the user automatically with an appropriate set of renderings of the deformation result covering the influence space of the deformation and making it thus obsolete to interactively post-explore the data to verify the results. Thus, it allows efficient object manipulation focusing the user on the desired manipulation and not on previously required manipulation preparation steps.

Applications and use of the above-described embodiments according to the invention are various and include exemplary fields such as interactive shape manipulation either per se (without an associated medical image), or in the context of shape to medical image adaptation (e.g. segmentation of anatomical objects in medical images). The method may be used for all 3D medical imaging modalities and to all procedures that require interactive initialization, correction, or verification of 3D or 4D shapes to image adaptation procedures.

According to an embodiment, the method is used to manipulate a 3D shape without any data context.

In an embodiment the 3D shape, before the manipulation, is a straight tube. Using the method, the straight tube is manipulated step by step to resemble an anatomical item (e.g. the aortic arch as drawn in an anatomical text book or as known by heart), e.g. by pulling and pushing on surface portions. In this case the manipulation scheme is the tube-bending scheme allowing reducing the degrees of freedom for the user, while there is no accompanying image or dataset. Moreover, a paddle-wheel visualization in this case could either show only the cut-lines of the shape object or not be utilized.

The invention may be implemented in any suitable form including hardware, software, firmware or any combination of these. However, preferably, the invention is implemented as computer software running on one or more data processors and/or digital signal processors. The elements and components of an embodiment may be physically, functionally and logically implemented in any suitable way. Indeed, the functionality may be implemented in a single unit, in a plurality of units or as part of other functional units. As such, the invention may be implemented in a single unit, or may be physically and functionally distributed between different units and processors.

Although the present invention has been described above with reference to specific embodiments, it is not intended to be limited to the specific form set forth herein. Rather, the invention is limited only by the accompanying claims and, other embodiments than those specified above are equally possible within the scope of these appended claims.

In the claims, the term "comprises/comprising" does not exclude the presence of other elements or steps. Furthermore, although individually listed, a plurality of units, elements or method steps may be implemented by e.g. a single unit or processor. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A method of manipulating a shape of a digital object, wherein said object is comprised in a dataset, the method comprising:
selecting an object to be manipulated;
interactively rendering said object in dependence of a manipulation type, wherein said rendering visualizes the impact of the shape manipulation in at least two view planes simultaneously, said at least two view planes intersecting each other in a line comprising at least one point of said shape manipulation.

2. The method according to claim 1, wherein said at least two view planes correspond to a paddle-wheel like set of at least two multi-planar image reformats (31a, 31b).

3. The method according to claim 2, wherein the position and axis of said paddle-wheel like set of at least two multi-planar image reformats is a selected point (32) and the surface normal (33) of said dataset shape, respectively.

4. The method according to claim 3, wherein the orientation of said paddle-wheel like set of at least two multi-planar image reformats is defined along the axis of a tubular structure.

5. The method according to claim 1, wherein said manipulation type is a manipulation of a surface patch (21) along a surface normal (22).

6. The method according to claim 1, wherein said manipulation type is a manipulation using two degrees of freedom, and wherein said two degrees of freedom correspond to the amount of push/pull and the radius of influence.

7. The method according to claim 1, wherein said manipulation type is a manipulation using two degrees of freedom, wherein said object is a tubular object, and wherein said two degrees of freedom correspond to the tube bending normal and the longitudinal tube axis.

8. The method according to claim 1, wherein said manipulation type is dependent on local shape properties.

9. The method according to claim 1, wherein said manipulation type is a manipulation based on a shape model.

10. The method according to claim 1, wherein said manipulation type is an object steered manipulation.

11. An apparatus for interactive digital shape manipulation for performing the method according to any one of claims 1-10, comprising:
a selection unit (41) for selecting an object to be manipulated; and
a rendering unit (42) for interactively rendering the object in dependence of a manipulation type, wherein said rendering visualizes the impact of the shape manipulation in at least two view planes simultaneously, said at least two view planes intersecting each other in a line comprising at least one point of said shape manipulation.

12. The apparatus according to claim 11, further comprising a display (43) for displaying the shape manipulation.

13. The apparatus according to claim 12, wherein said apparatus is included in a medical workstation.

14. A computer-readable medium having embodied thereon a computer program (50) for processing by a computer, the computer program (50) comprising:
a shape manipulation code segment (51) for manipulating a shape of an object,
a render code segment (52) for interactively rendering said object in dependence of a manipulation type, wherein said rendering visualizes the impact of the shape manipulation in at least two view planes simultaneously, said at least two view planes intersecting each other in a line comprising at least one point of said shape manipulation.

15. The computer-readable medium according to claim 14 comprising code segments arranged, when run by an apparatus having computer processing properties, for performing all of the method steps defined in any one of claims 1-10.
